# EUROPEAN PATENT APPLICATION

(11) **EP 2 196 445 A1**
(43) Date of publication of application: **16.06.2010**
(21) Application number: 08171638.3
(22) Date of filing: 15.12.2008
(51) Int. Cl.: C07C 17/20, C07C 17/42, C07C 19/08, C07B 63/04

(54) **Process for reducing the metal content in organic heavy boiler waste**

(71) Applicant: Solvay Fluor GmbH, 30173 Hannover (DE)
(72) Inventor: Uenveren, Ercan, 30625 Hannover (DE); Eichholz, Kerstin, 30855 Langenhagen (DE); Eicher, Johannes, 31319 Sehnde (DE); Lambert, Alain, 1320 Beauvechain (BE)
(74) Representative: Mross, Stefan P.M.

(57) **Abstract**

The invention relates to a process for reducing the metal content in metal-containing organic heavy boiler waste comprising adding a base to the metal-containing organic heavy boiler waste thereby forming a phase which contains the organic boiler waste with a reduced metal content and at least one additional phase and separating the phase which contains the heavy boiler waste and has a reduced metal content.

## Description

The invention relates to a process for reducing the metal content in metal-containing organic heavy boiler waste which is produced as a by-product e.g. in a process for producing fluorinated hydrocarbons in the presence of a metal-containing catalyst. The invention thus also relates to a process for producing fluorinated hydrocarbons in the presence of a metal-containing catalyst, which process comprises a process step in which the metal content of the organic heavy boiler waste is reduced.

The fluorination process of organic compounds has been well developed during many years. Generally, in such a process chlorinated and/or unsaturated hydrocarbons are treated with HF under elevated pressure and temperature in the presence of a metal-containing catalyst. The catalyst usually contains metal halides, e.g. chlorides and fluorides of transition metals and metals of the main groups of the periodic system.

In such a process generally undesired organic heavy boiler waste is formed as by-product. Therefore, whether the process is a batch process or a continuous process, the reaction vessel has to be purged periodically to maintain an adequate catalyst activity and finally to ensure an efficient process.

The formed undesired heavy boiler waste has to be disposed of. The heavy boiler waste contains organic compounds having higher molecular weights, and an easy way to dispose of this heavy boiler waste would be by incineration. However, the heavy boiler waste contains catalyst or degradation products from the catalyst, i.e. metal components. The metal components of the catalyst are usually environmentally problematic and are not rendered harmless by usual incineration. Therefore, depending on the amount of metal components in the heavy boiler waste, it is not admissible to simply bum the heavy boiler waste for environmental concerns. Especially, environmentally safe burning processes using sophisticated filter techniques and/or burners are required. This significantly increases the costs of disposing the heavy boiler waste.

It would therefore be a great economic and environmental advantage, if the metal could be removed from the heavy boiler waste, preferably completely, however, any reduction of the metal content of the heavy boiler waste would also be advantageous, because heavy boiler waste with a reduced metal content can be disposed of with lower costs.

EP 0 522 639 discloses a process for the manufacture of 1-chloro-1,1,3,3,3-pentafluoropropane. In this process the chlorinated precursor 1,1,1,3,3,3-hexachloropropane is treated with HF at a temperature of about 100°C and in the presence of catalysts which contain metals from the groups IIIa, IVa, IVb, Va, Vb and VIb of the periodic table of elements. The preferred catalysts are chlorides and fluorides of titanium, tantalum, molybdenum, tin and antimony. All those metals are highly problematic and heavy boiler waste with those metals can only be disposed of by incineration if the content of the metals in the heavy boiler waste is low.

US 5,739,406 discloses a process for the hydrofluorination of chloro(fluoro)butanes in the presence of a catalyst chosen from derivatives of metals of groups IIIa, IVa, IVb, Va, Vb and VIb of the periodic table of the elements.

US-A 5,894,075 discloses a process for producing organic compounds containing at least one fluorine atom, comprising introducing at least one fluorine atom by metal halide catalyzed addition of HF to an unsaturated C-C group or by metal halide catalyzed chlorine-fluorine exchange of HF with at least one fully halogenated carbon atom in a starting material, wherein the introducing step is carried out in the presence of a metal halide catalyst comprising an at least partially fluorinated mixture of titanium chloride or titanium bromide in combination with at least partially fluorinated tin tetrachloride or tin tetrabromide in which titanium and tin are present in a certain atomic ratio.

The above documents are cited as examples of the large number of documents which deal with processes in which unsaturated hydrocarbons or fluorinated hydrocarbons are treated with HF in the presence of metal-containing catalysts in order to prepare fluorohydrocarbons (which may contain in addition one or more chlorine atoms). In all those processes high molecular weight organic by-products are produced which in the art are generally termed "heavy boiler waste" and which cannot be recycled to the process and must therefore be removed from the process and disposed of. In all those processes and in all other processes of the prior art which treat (chloro)hydrocarbons with hydrofluoric acid in the presence of a metal-containing catalyst it would be advantageous, if the metal content of the heavy boiler waste could be reduced in order to avoid problems which occur if heavy boiler waste with a high metal content is to be disposed of.

Therefore, it would be highly advantageous, if a possibility existed to reduce the metal content in heavy boiler waste, and such a process could preferably be combined with a process for producing fluorinated hydrocarbons with hydrofluoric acid in the presence of metal catalysts.

The present inventors now found that it is easily and economically possible to reduce the metal content in the heavy boiler waste by treating the heavy boiler waste with a base.

The present invention therefore provides a process for reducing the metal content in metal-containing organic heavy boiler waste comprising adding a base to the metal-containing organic heavy boiler waste, thereby forming a phase which contains the organic heavy boiler waste having a reduced metal content and at least one additional phase and separating the phase which contains the organic heavy boiler waste having a reduced metal content.

The present invention also provides a process for producing fluorinated hydrocarbons optionally substituted with one or more chlorine atoms, comprising
(i) treating hydrochlorocarbons, optionally substituted with one or more fluorine atoms with HF in the presence of a metal-containing catalyst,
(ii) separating the organic heavy boiler waste that is produced in process step (i) and contains metal impurities,
(iii) adding a base to the metal-containing organic heavy boiler waste, thereby forming a phase which contains the organic heavy boiler waste with a reduced metal content and at least one additional phase and
(iv) separating the phase containing the organic heavy boiler waste with a reduced amount of metal.

Treating the organic heavy boiler waste with a base removes at least a part of the metal in the heavy boiler waste that usually origins from the metal-containing catalyst used in the reaction which produced the heavy boiler waste. It is believed that the base forms a reaction product with the metal-containing impurities. This reaction product can be present in a solid phase which is a precipitate which contains at least a part of the metal. It is also possible that a further (liquid) phase is present which contains a part of the metal, in particular, if the base is used in the form of an aqueous solution (see below). The precipitate (solid phase) or the metal-containing additional (preferably aqueous) phase can easily be separated from the liquid phase which contains the heavy boiler waste and can then be easily disposed of, while the retaining heavy boiler waste contains only a reduced amount of metal or ideally (e.g. after repeated treatments) no metal at all. This organic heavy boiler waste after treatment can then be burned in a cheaper and more environmentally friendly way. Additionally, the base neutralizes HF and other acids which can still be present in the heavy boiler waste.

The process of the present invention for reducing the metal content in metal-containing organic heavy boiler waste can be applied to any metal-containing organic heavy boiler waste. This waste is usually produced by treating a suitable reagent with hydrofluoric acid (HF) in the presence of a metal-containing catalyst to produce fluorinated hydrocarbons, and therefore, the process of the present invention is particularly useful in connection with such a process. However, the process of the present invention can also be used with metal-containing organic heavy boiler waste that is produced as a by-product in other reactions.

Therefore, in a preferred embodiment the process of the present invention for reducing the metal content of metal-containing organic heavy boiler waste is part of a process for producing fluorinated hydrocarbons by treating a suitable reagent with hydrofluoric acid in the presence of a metal-containing catalyst. Such processes are also part of the present invention. The processes for producing fluorinated hydrocarbons by treating a reagent with hydrofluoric acid in the presence of a metal catalyst are not particularly limited, as all known processes in which a reagent is treated with hydrofluoric acid in the presence of a metal catalyst to produce fluorinated hydrocarbons also produce metal-containing organic heavy boiler waste that can advantageously be processed according to the present invention. As examples of typical processes for producing fluorinated hydrocarbons, the processes disclosed in the documents discussed in the prior art section of this specification (EP 0 522 639, US 5,739, 406 and US 5,894, 075) can be mentioned, however, many other processes are known to a skilled person. The reagent used in those processes, which is treated with hydrofluoric acid in the presence of a metal-containing catalyst, is generally a hydrochlorocarbon that can already be substituted with one or more fluorine atoms. With those reagents either a chlorine atom is substituted with a fluorine atom or hydrofluoric acid is chemically added to an olefinic bond (double bond). Thus, the fluorinated hydrocarbons obtained by the process of the invention can optionally contain also chloro atoms, and they can be either saturated or unsaturated. Neither the reagents nor the end products are particularly restricted and are known to a person skilled in the art.

As example of fluorinated products to be produced by the process of the present invention products of the general formula I can be mentioned:

**CₙHₘClₒFₚ**

wherein
n is an integer of 1 to 8
m is an integer of 1 to 16
o is an integer of 0 to 10
p is an integer of 2 to 16
m + o + p = 2 n + r and
r is 0, -2 or 2.

As will be understood by a skilled person the above formula is to be interpreted that the indices n, m, o and p cannot be freely selected within the indicated ranges but only those selections are possible which meet the criteria m + o + p = 2 n + r. If r = 2, it means that the compounds are saturated. In the case that r = 0 or -2 the compounds contain one or two double bonds, respectively. In case where the compounds contain one double bond of course n must be at least 2 and in case that the compounds contain two double bonds, n must be at least 4. Of course, the corresponding reagents either have at least one chlorine atom more than the above end products of formula I (which chlorine is then replaced by a fluorine atom during the reaction with hydrofluoric acid in the presence of a metal-containing catalyst) or they contain at least one additional unsaturation (either an additional olefinic bond or a triple bond) to which the hydrofluoric acid is chemically added.

Most preferably, the process according to the invention is a process for the manufacture of 1,1-Difluoroethane, 1,1,1-Trifluoroethane, 1,1-Difluoro-1-chloroethane, 1,1,1,3,3-Pentafluoropropane, 1,1,1,3,3,3-Hexafluoropropane, 1,1,1,3,3-Pentafluorobutane or 1,1,1,2,3,4,4,5,5,5-Decafluoropentane.

The catalysts used in the process of the invention are not particularly limited, however, they are always metal-containing catalysts. Generally, the metal-containing catalysts contain metals of the groups IIIa, IVa, IVb, Va, Vb and/or VIb of the periodic table of elements, preferably titanium, tantalum, molybdenum, tin and/or antimony, more preferably titanium, tin and/or antimony, still more preferably titanium or tin or a mixture of both, and most preferably the metal catalysts contain titanium as catalytically active metal, preferably as sole catalytically active metal.

The metal-containing catalyst is very often a metal halide, and the preferred halides are chlorides and fluorides or partially fluorinated chlorides or mixtures thereof. Thus, the preferred metal-containing catalysts are e.g. titanium fluoride, titanium chloride, tin fluoride, tin chloride, titanium or tin mixed chlorides and fluorides and most preferably titanium fluoride.

In the process for producing fluorinated hydrocarbons generally a mixture of heavy boiler waste and light boiler waste is obtained as is known in the art. The heavy boiler waste is generally separated from the light boiler waste by known methods (e.g. by evaporation under elevated temperature and optionally reduced pressure).

Heavy boiler waste is a technical term that is well understood and clear in the technical field. Heavy boiler waste means organic compounds with a boiling point significantly higher than the end product of the industrial process in which the heavy boiler waste is produced. In particular, because of its high boiling point, heavy boiler waste cannot be recycled to the process and must therefore be disposed of. "Significantly higher" in this context means that the boiling point of the organic compounds at atmospheric pressure is generally more than 30°C, usually more than 40°C higher than the boiling point of the end product.

The exact composition of the heavy boiler waste depends on the process in which it is produced, but for example, in the most preferred process of the present application, which is the process for producing HFC-365mfc (1,1,1,3,3-Pentafluorobutane), the boiling point of the end product HFC-365mfc is about 40°C, and heavy boiler waste includes all organic compounds that cannot be recycled and have a boiling point of 70°C or more.

In the processes of the present invention (both the process for reducing the metal content in metal-containing organic heavy boiler waste and in the process for producing fluorinated hydrocarbons) the base can be added as such to the organic heavy boiler waste. However, very often the organic heavy boiler waste is highly viscous, and thus it is preferred that one or more organic solvents are added to the organic heavy boiler waste (thereby diluting the heavy boiler waste) before the base is added or together with the base.

In one embodiment of the present invention, the heavy boiler waste still contains a significant amount of the light boilers from the fluorination reaction and in this embodiment it is generally not necessary to add additional solvent.

The solvents added to the heavy boiler waste are standard organic solvents known by a person skilled in the art preferably with a boiling point of less than 100°C but not lower than 30°C, such as acetone, dichloromethane (DCM), chloroform and tetrachloromethane, preferred is dichloromethane.

The ratio of heavy boiler waste and organic solvents to be added is not particularly restricted but is generally between 0.25 and 2, preferably 0.5 and 1 (on weight basis). Of course, the ideal amount of solvent to be added depends on the viscosity of the heavy boiler waste and the solvent and can vary from one batch to the next. The finding of an acceptable kind and amount of solvent to be added belongs to the general skills of a chemist.

For the processes of the present invention it is essential that a base is added to the metal-containing organic heavy boiler waste. The base is not particularly restricted and can be added to the heavy boiler waste in many different ways. For example, the base can be added as such, which is particularly preferred, if the base is gaseous or liquid. However, the base can also be added to the heavy boiler waste in the form of a solution or suspension, preferably an aqueous solution or aqueous suspension, however, an organic solution or organic suspension is also preferred.

The term "adding a base to the metal-containing organic heavy boiler waste" should be interpreted broadly as mixing the base with the metal-containing organic heavy boiler waste. The addition can be effected by adding the base and the metal-containing organic heavy boiler waste at the same time into a vessel, by providing a vessel containing the base and adding the metal-containing organic heavy boiler waste into the vessel or by providing a vessel containing the metal-containing organic heavy boiler waste and adding the base to the vessel. In practice, generally the metal-containing organic heavy boiler waste is present in a vessel to which the base is added.

In the same way, the term "adding an organic solvent to the metal-containing organic heavy boiler waste" is to be interpreted broadly as mixing an organic solvent with the metal-containing organic heavy boiler waste.

If the base is added to the heavy boiler waste together with an organic solvent, e.g. in the form of a solution in an organic solvent or suspension in an organic solvent, the organic solvent in which the base is used can be the organic solvent which dilutes the heavy boiler waste. In this case no additional separate addition of solvents for diluting the heavy boiler waste may be necessary, but the organic solvent that is introduced together with the base may take the function of the organic solvent for diluting the heavy boiler waste. When in the present specification it is disclosed that the heavy boiler waste is diluted with an organic solvent, it should be understood that this does not require an independent process step but that the step of diluting the heavy boiler waste with a solvent and the step of adding the base can be done at the same time, if the base is added together with an organic solvent. However, it is preferred that the organic solvent is added to the heavy boiler waste before the base is added.

If the base is added to the heavy boiler waste as such or together with an organic solvent, it is possible that only one liquid phase forms, and in this case a solid phase, i.e. a precipitate will form within this one liquid phase which contains at least a part of the metal from the heavy boiler waste. The precipitate can then be separated and disposed of, the remaining solution of heavy boiler waste in an organic solvent can be further processed by removing the organic solvent (e.g. by evaporation). This results in heavy boiler waste with a reduced amount of metal, which can then be disposed of in a more environmentally friendly and cheaper way than before.

It is also possible that after the base has been added to the heavy boiler waste a separate liquid phase forms, e.g. if the solvent used for adding the base is not miscible with the heavy boiler waste, e.g. if an aqueous base system is employed. In this case a precipitate may form or no precipitate may form, as discussed above, but the liquid phase that does not contain the heavy boiler waste contains at least a part of the metal impurities. This phase can be separated, and the liquid phase with the heavy boiler waste can then be further processed, as discussed above, to obtain the heavy boiler waste with reduced amount of metal.

If the base is added as such, e.g. as a gas or as a solid or as a liquid, the heavy boiler waste is preferably diluted with an organic solvent before the addition of the base to form a liquid phase. In this case generally a precipitate (solid phase) forms which is enriched in the metal, and this precipitate can be separated from the organic solution containing the heavy boiler waste. Again the solution containing the heavy boiler waste can be further processed to remove the solvent, and the remaining heavy boiler waste is reduced in metal content.

In a most preferred embodiment of the present invention first an organic solvent is added to the heavy boiler waste, and then an aqueous system containing the base is added. The aqueous system can either be a solution of the base in water or a suspension of the base in water, preferably a solution of the base in water. In this case two liquid phases are generally formed, an aqueous phase and an organic phase, the aqueous phase containing at least a part of the metal component, while the organic heavy boiler waste remains in the organic phase. The aqueous phase can be separated, the organic phase containing a solvent and the heavy boiler waste can be further processed to separate the heavy boiler waste with reduced amount of metal. Of course, in this case, additionally to the aqueous phase, a precipitate can be formed which also contains metal, and this precipitate (i.e. a solid phase) can also be separated from the organic phase containing the heavy boiler waste leading to a further reduction in the metal content of the heavy boiler waste.

Preferred bases for adding to the heavy boiler waste according to the invention are ammonia (NH₃) which can either be added as a gas stream or as an aqueous solution. Furthermore preferred are organic bases, in particular organic amines, more preferably aliphatic amines, still more preferably aliphatic amines with 1 to 20 carbon atoms. The aliphatic amines can be primary, secondary or tertiary amines, such as isopropyl amine, tert-propyl amine, n-propyl amine or tributyl amine, and those amines are generally used in the form of a solution in an organic solvent such as acetone or any of the other organic solvents mentioned above used for diluting the heavy boiler waste. Of course, according to the invention it is possible to add more than one base, e.g. a mixture of bases, and the term "adding a base" does not exclude that several bases are added. Most preferred are aqueous ammonia and isopropyl amine in a suitable solvent such as acetone.

The amount of base added to the organic heavy boiler waste is not particularly limited and depends on the kind of base, the heavy boiler waste and the type and amount of impurities in the heavy boiler waste and the amount of eventually present acids, like e.g. hydrofluoric acid, in the heavy boiler waste. Apart from the amount of base which is necessary for neutralizing the present acids, a minimum of one mole of base should be added per mole of metal atoms present in the heavy boiler waste. The molar ratio between base to be added and metal atoms present in the heavy boiler waste is preferably in the range of 1:10, more preferably in the range of 1:8, more preferably in the range of 1:6 and in particular in the range of 1:4. However, it is not disadvantageous to add an excess amount of base, since any excess base can be recycled, and therefore, in general an amount of base is used which is in the range of 5% to 300%, more preferably of 5% to 200%, more preferably of 10% to 100% of the amount of heavy boiler waste (on a weight basis). With such a high amount of base, it is assumed that sufficient base is used to provide the molar ratios of base to metal mentioned above, even if some base is consumed by acids in the heavy boiler waste.

The metal-containing organic heavy boiler waste can either be formed in a batch process or as a stream in a continuous process. Both embodiments are within the present invention. According to the present invention the base is generally added to the heavy boiler waste in a suitable vessel (reactor) at a suitable temperature, e.g. room temperature (20°C to 25°C) or at slightly elevated temperatures (25°C to 60°C) taking care that the heavy boiler waste is carefully mixed with the base. However, the addition can also be carried out at higher temperatures, but this might be disadvantageous, because generally, the temperature increases when the base is added to the heavy boiler waste. If necessary, cooling can be applied to achieve the above temperature ranges. The mixing of the organic heavy boiler waste with the base can be done by any suitable mixing device known in the art, e.g. by stirring.

The process of the present invention for reducing the metal content in metal-containing organic heavy boiler waste can easily be combined with known industrial processes for producing fluorinated hydrocarbons. Generally, such an industrial process comprises a separation step after the reaction in which the fluorinated hydrocarbon is produced, wherein the reaction mixture is transferred to an evaporator to separate light boiler products from heavy boiler waste. In a preferred process of the present invention the following steps are added to such a known industrial process for preparing fluorinated hydrocarbons:
(i) To the stream of metal-containing organic heavy boiler waste withdrawn from the bottom of the evaporator in an industrial process for preparing fluorinated hydrocarbons optionally a solvent is added to dilute the metal-containing heavy boiler waste. The optionally diluted heavy boiler waste is sent to a vessel, where the base in the form of an aqueous solution is carefully added. The vessel is stirred and preferably cooled. A liquid aqueous phase, a liquid organic phase and, depending on the system, a solid phase are formed.
(ii) After this treatment the mixture is filtered in order to remove any solid phase.
(iii) The mixture is then introduced to a phase separator, where the liquid aqueous phase is separated, and the liquid organic phase is sent to an evaporator for recycling the solvent (preferably DCM). From the bottom of the evaporator the organic heavy boiler waste reduced in metal (preferably titanium) is obtained and can be sent to the incinerator.
(iv) The aqueous phase obtained from the phase separator can directly be recycled without any further treatment or the metal can be first removed.
(v) The excess base and the solvent are recycled.

The following examples further explain the invention.

### EXAMPLES

### Example 1

A stream of reaction mixture was taken from a reactor in which 1,1,1,3,3-pentafluorobutane is produced by treating a mixture of different 1,1,1,3,3-pentahalobutanes primarily comprising 3-chloro-1,1,1,3-tetrafluorobutane, 1-chloro-1,1,3,3-tetrafluorobutane, 1,1-dichloro-1,3,3-trifluorobutane, 1,3-difluoro-1,1,3-trichlorobutane, 3,3-difluoro-1,1,1-trichlorobutane, 3-fluoro-1,1,1,3-tetrachlorobutane and 1,1,1,3,3-pentachlorobutane with hydrofluoric acid in the presence of titaniumtetrafluoride as a catalyst (Ti concentration about 2.52%.

From the reaction mixture (containing the heavy boiler waste and all other components of the reaction) a 75 g sample was taken which was treated with 500 g 13% aqueous ammonia.

Treating the 75 g sample with the 500 g 13% aqueous ammonia resulted in two separate liquid phases, and in addition a 6.5 g solid phase (precipitate) occurred. The titanium was significantly enriched in the precipitate and in the aqueous phase, the organic phase containing the heavy boiler waste contained only a very small amount of titanium (17 ppm Ti in the aqueous phase, 29% Ti in the precipitate and only 7 ppm Ti in the organic phase.

Another 100 g sample was taken and treated with a solution of 60 g isopropyl amine in 400 g acetone. Treating the sample with a solution of isopropyl amine in acetone resulted in one liquid phase containing the heavy boiler waste and one solid phase (precipitate) that contains a significant amount of titanium (2000 ppm in the precipitate, 98 ppm in the organic phase).

As a comparative example a sample was treated with aqueous hydrochloric acid, and no phase separation could be seen. It was not possible to remove titanium from the reaction mixture.

### Example 2

In this example a further sample was taken as in example 1 but light boilers were first evaporated from the reaction mixture. The heavy boiler waste remained which contained the catalyst metal and a significant amount of acids (e.g. HF).

A 30 g sample and three 20 g samples were taken from the heavy boiler waste. The 30 g sample was diluted with the same amount of DCM, and 0.1 mole of tert-butyl amine was added. A solid phase was obtained that was enriched in titanium and could easily be separated by filtration. The solid phase (precipitate) was washed with acetone, and a white precipitate with very high titanium content was obtained.

The three 20 g samples of the heavy boiler waste were not diluted with DCM but treated with hydrochloride acid, aqueous ammonia and a mixture of isopropyl amine in acetone. The results are shown in the following table.

| **Base / Acid** | **Amount (g)** | **Organic (g)** | **Phase Separation** | **Results (Ti %)** |
|---|---|---|---|---|
| t-BuAm | 7,3 | 30 | Solid phase | One solid phase 2.65%; after washing with acetone white precipitate was obtained 5.59 % |
| HCl 32% | 30 | 20 | No phase separation | 1.7% |
| NH₃ 13% | 20 | 20 | Good phase separation /solid - aqueous - organic | 0.28% in solid, 1.38% in aqueous phase, 0.1% in organic phase |
| Acetone / IPA | 15/6 | 20 | Precipitation | 0.42% in solid, 0.26% in liquid |

After evaluation of the results it was observed that the Ti amounts of the taken portions somewhat differ from each other. It appears that in the highly viscous heavy boiler waste the titanium is not distributed homogeneously.

As shown in the table, no phase separation occurred in the hydrochloric acid experiment. Thus, the amount of Ti could not be reduced in the heavy boiler waste.

In the ammonia example it can be clearly seen that the Ti concentration in the organic phase was reduced. Presumably, most of the Ti amount was transferred to the aqueous phase. A small part of the solid phase could be separated from the aqueous phase using regular filter paper.

In the acetone / IPA example it is seen that the Ti concentration is relatively reduced.

### Example 3

Again, a sample from the reaction mixture was taken as in example 1. The light boilers were first evaporated from the reaction mixture. The heavy boiler waste remained which contained the catalyst metal and a significant amount of acids (e.g. HF). Then a portion of this heavy boiler waste was diluted with an organic solvent.

In particular, four samples of 25 g were taken from the very viscous heavy boiler waste. To each sample dichloromethane (DCM) or tetrachloromethane (CCl₄) with a weight ratio of 1:1 or 1:2 (organic waste: solvent) was added. Then 40 g 13% aqueous ammonia was injected slowly to each mixture during about 14 hours (because of the highly exothermic reaction). A three-phase system developed, an aqueous phase, an organic phase and a solid precipitate. The mixture was filtered with paper filter (4-12 µm pore size, medium speed Weißbandfilter 589/2) for removing the solid, and the aqueous phase was removed using a separation funnel. The results are summarized in the following tables:

**Experiment WO:DCM = 1:1**

| | all | Ti conc. | Ti amount |
|---|---|---|---|
| | g | % | g |
| Waste organic (WO) | 25 | 3.07 | 0.768 |
| DCM | 25 | | |
| 13% NH₃ (aq) | 40 | | |
| Total weight after reaction | 79 | | |
| Weight loss during reaction | 11 | | |
| Solid phase | 4.2 | 18.02 | 0.757 |
| Aqueous phase | 34.6 | 0.026 | 0.0090 |
| Organic phase | 40.2 | 0.005 | 0.0020 |

**Experiment WO:DCM = 1:2**

| | all | Ti conc. | Ti amount |
|---|---|---|---|
| | g | % | g |
| Waste organic (WO) | 25 | 2.72 | 0.681 |
| DCM | 50 | | |
| 13% NH₃ (aq) | 40 | | |
| Total weight after reaction | 97.6 | | |
| Weight loss during reaction | 17.4 | | |
| Solid phase | 4.1 | 16.40 | 0.672 |
| Aqueous phase | 34.8 | 0.024 | 0.0084 |
| Organic phase | 58.7 | 0.001 | 0.0006 |

**Experiment WO:CCl₄ = 1:1**

| | all | Ti conc. | Ti amount |
|---|---|---|---|
| | g | % | g |
| Waste organic (WO) | 25 | 2.82 | 0.705 |
| CCl₄ | 25 | | |
| 13% NH₃ (aq) | 40 | | |
| Total weight after reaction | 82,9 | | |
| Weight loss during reaction | 7.1 | | |
| Solid phase | 4 | 17.41 | 0.696 |
| Aqueous phase | 34.8 | 0.023 | 0.0080 |
| Organic phase | 44.1 | 0.002 | 0.0009 |

**Table 4: Experiment WO:CCl₄ = 1:2 4**

| | all | Ti conc. | Ti amount |
|---|---|---|---|
| | g | % | g |
| Waste organic (WO) | 25 | 1.74 | 0.434 |
| CCl₄ | 50 | | |
| 13% NH₃ (aq) | 40 | | |
| Total weight after reaction | 105.1 | | |
| Weight loss during reaction | 9.9 | | |
| Solid phase | 2.5 | 16.59 | 0.415 |
| Aqueous phase | 39.2 | 0.045 | 0.0176 |
| Organic phase | 63.4 | 0.003 | 0.0019 |

The addition of DCM or CCl₄ reduced the viscosity of the organic heavy boiler waste which contained Ti. The ammonia treatment lead to a precipitate which could be removed by filtration in the experiments so that the Ti amount in the remaining liquid organic and aqueous phases was significantly reduced. When DCM was used a material loss was observed probably due to its low boiling point (40°C). In the experiments open systems were used for preventing pressure increase in the system. When both types of systems are compared regarding phase separation it was observed that DCM forms one proper organic phase as CCl₄ tends to form two organic phases. Additionally, the recovery of DCM should be easier than the one of CCl₄ because of its lower boiling point. Moreover, CCl₄ is a compound more expensive and toxic compared with DCM and therefore DCM is more favorable.

## Claims

1. Process for reducing the metal content in metal-containing organic heavy boiler waste comprising adding a base to the metal-containing organic heavy boiler waste thereby forming a phase which contains the organic boilers with a reduced metal content and at least one additional phase and separating the phase which contains the heavy boiler waste and has a reduced metal content.

2. Process according to claim 1 comprising adding an organic solvent to the metal-containing organic heavy boiler waste before or together with the base.

3. Process according to claim 2 comprising separating an organic phase after adding the base to the metal-containing organic heavy boiler waste and recovering at least a part of the organic solvent from the organic phase.

4. Process according to any of the preceding claims, wherein the organic heavy boiler waste is a by-product from a process for producing fluorinated hydrocarbons using hydrofluoric acid in the presence of a metal-containing catalyst.

5. Process according to claim 4, wherein the fluorinated hydrocarbons are selected from the group consisting of 1,1-difluoroethane, 1,1,1-trifluoroethane, 1,1-difluoro-1-chloroethane, 1,1,1,3,3-pentafluoropropane, 1,1,1,3,3,3-hexafluoropropane, 1,1,1,3,3-pentafluorobutane or 1,1,1,2,3,4,4,5,5,5-decafluoropentane.

6. Process according to any of the preceding claims, wherein the metal is selected from the group consisting of titanium, antimony and tin.

7. Process according to any of claims 2 to 6, wherein the organic solvent is selected from the group consisting of acetone, dichloromethane, chloroform and tetrachloromethane.

8. Process according to any of claims 2 to 7, wherein the weight ratio of heavy boiler waste to organic solvent is in the range of 0.25 to 2.

9. Process according to any of the preceding claims, wherein the base is selected from the group consisting of gaseous NH₃, aqueous NH₃ and organic amines.

10. Process for producing fluorinated hydrocarbons optionally substituted with one or more chlorine atoms, comprising
(i) treating hydrochlorocarbons, optionally substituted with one or more fluorine atoms with HF in the presence of a metal-containing catalyst,
(ii) separating the organic heavy boiler waste that is produced in process step (i) and contains metal impurities,
(iii) adding a base to the metal-containing organic heavy boiler waste, thereby forming a phase which contains the organic heavy boiler waste with a reduced metal content and at least one additional phase,
(iv) separating the phase containing the organic heavy boiler waste with a reduced amount of metal.

11. Process according to claim 10 comprising the additional step of adding an organic solvent to the metal-containing organic heavy boiler waste from process step (ii) and recovering at least a part of the organic solvent after step (iv).

12. Process according to claim 10 or 11, wherein the fluorinated hydrocarbons are selected from the group consisting of 1,1-difluoroethane, 1,1,1-trifluoroethane, 1,1-difluoro-1-chloroethane, 1,1,1,3,3-pentafluoropropane, 1,1,1,3,3,3-hexafluoropropane, 1,1,1,3,3-pentafluorobutane or 1,1,1,2,3,4,4,5,5,5-decafluoropentane.

13. Process according to any of claims 10 to 12, wherein the metal-containing catalyst contains a metal selected from the group consisting of titanium, antimony and tin.

14. Process according to any of claims 11 to 13, wherein the organic solvent is selected from the group consisting of acetone, dichloromethane, chloroform and tetrachloromethane.

15. Process according to any of claims 11 to 14, wherein the weight ratio of heavy boiler waste to organic solvent is in the range of 0.25 to 2.

16. Process according to any of claims 10 to 15, wherein the base is selected from the group consisting of gaseous NH₃, aqueous NH₃ and organic amines.
